# EUROPEAN PATENT APPLICATION

(11) **EP 4 726 734 A1**
(43) Date of publication of application: **15.04.2026**
(21) Application number: 24206127.3
(22) Date of filing: 11.10.2024
(51) Int. Cl.: G16H 40/40

(54) **A SYSTEM AND METHOD FOR GENERATING A DEVICE DRIVER FOR A MEDICAL DEVICE**

(71) Applicant: Radiometer Medical ApS, 2700 Brønshøj (DK)
(72) Inventor: SØDERHAMN MIDTGAARD, Michael, 2700 Brønshøj (DK); LIU, Wei, 2700 Brønshøj (DK); MAJGÅRD, Per, 2700 Brønshøj (DK)
(74) Representative: 2SPL Patentanwälte PartG mbB

(57) **Abstract**

A system comprises one or more processors and one or more storage devices, wherein the system is configured to receive a communication request from a medical device; transmit a message of a message type to the medical device according to a first message scheme for the message type using a first device driver; receive an error message from the medical device in response to the message; select a second message scheme for the message type from a plurality of message schemes stored by the one or more storage devices; and generate a second device driver based on the first device driver and the second message scheme.

## Description

### Technical field

Examples relate to the connectivity of medical devices.

### Background

Connectivity of medical devices (e.g. Point-of Care Testing Devices; POCT) to middleware systems is required in order to manage quality, accreditation, and compliance in an efficient manner. Users also require efficient and fast turn-around time for adding new as well as updating existing connectivity to improve the clinical use of POCT devices.

The development of drivers which enable connectivity to, e.g., POCT devices is currently a time-consuming and cumbersome process.

There is therefore a need to improve the turn-around time and efficiency for development of drivers for medical devices that need to be connected.

### Summary

This demand may be met by a system and a method for generating a device driver for a medical device and a computer program in accordance with the claims.

An example relates to a system comprising one or more processors and one or more storage devices. The system is configured to receive a communication request or a response to a communication request from a medical device and transmit a message of a message type to the medical device according to a first message scheme for the message type using a first device driver. Further, the system is configured to receive an error message from the medical device in response to the message and select a second message scheme for the message type from a plurality of message schemes stored by the one or more storage devices. Additionally, the system is configured to generate a second device driver based on the first device driver and the second message scheme.

A further example relates to a computer-implemented method for generating a device driver for a medical device. The method comprises receiving a communication request or a response to a communication request from a medical device and transmitting a message of a message type to the medical device according to a first message scheme for the message type using a first device driver. Further, the method comprises receiving an error message from the medical device in response to the message and selecting a second message scheme for the message type from a plurality of message schemes. Additionally, the method comprises generating a second device driver based on the first device driver and the second message scheme.

Another example relates to a system comprising one or more processors and one or more storage devices. The system is configured to receive device information of a medical device and provide a message of a message type to a device simulator according to a first message scheme for the message type using a first device driver. Further, the system is configured to simulate, by the device simulator based on the device information, a first response of the medical device to the message transmitted according to the first message scheme. Additionally, the system is configured to select a second message scheme for the message type from a plurality of message schemes stored by the one or more storage devices, if the first response indicates an error. Furthermore, the system is configured to generate a second device driver based on the first device driver and the second message scheme and provide a message of the message type to the device simulator according to the second message scheme for the message type using the second device driver. Additionally, the system is configured to simulate, by the device simulator based on the device information, a second response of the medical device to the message transmitted according to the second message scheme.

A further example relates to a computer-implemented method for generating a device driver for a medical device. The method comprises receiving device information from a medical device and providing a message of a message type to a device simulator according to a first message scheme for the message type using a first device driver. Further, the method comprises simulating, by the device simulator based on the device information, a first response of the medical device to the message transmitted according to the first message scheme. Additionally, the method comprises selecting a second message scheme for the message type from a plurality of message schemes, if the first response indicates an error. Furthermore, the method comprises generating a second device driver based on the first device driver and the second message scheme and providing a message of the message type to the device simulator according to the second message scheme for the message type using the second device driver. Additionally, the method comprises simulating, by the device simulator based on the device information, a second response of the medical device to the message transmitted according to the second message scheme.

### Short description of the Figures

Some examples of apparatuses and/or methods will be described in the following by way of example only, and with reference to the accompanying figures, in which
Fig. 1 shows a schematic illustration of a system for generating a device driver;
Fig. 2 shows a flow chart of a message flow between a medical device and a host;
Fig. 3 shows a schematic illustration of a system for generating a device driver;
Fig. 4 shows a schematic illustration of a system for generating a device driver;
Fig. 5a shows a table of a part of a topic registry;
Fig. 5b shows a table of a part of a topic registry;
Fig. 6a shows a schematic illustration of hardcoded drivers;
Fig. 6b shows a schematic illustration of drivers using a topic registry;
Fig. 7 shows a flow chart of a method for generating a device driver for a medical device;
Fig. 8 shows a schematic illustration of a system for generating a device driver; and
Fig. 9 shows a flow chart of a method for generating a device driver for a medical device.

### Detailed Description

Various examples will now be described more fully with reference to the accompanying drawings in which some examples are illustrated. In the figures, the thicknesses of lines, layers and/or regions may be exaggerated for clarity.

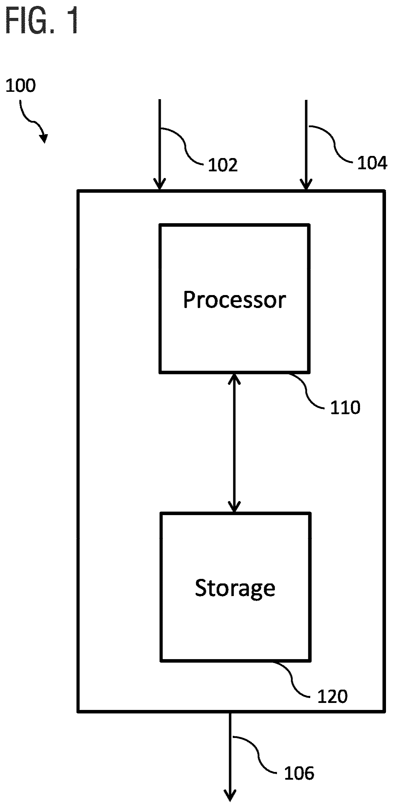

Fig.1 shows a system 100 for generating a device driver. The system 100 comprises one or more processors 110 and one or more storage devices 120. The system 100 is configured to receive a communication request 102 or a response to a communication request from a medical device and transmit a message 106 of a message type to the medical device according to a first message scheme for the message type using a first device driver. Further, the system 100 is configured to receive an error message 104 from the medical device in response to the message 106 and select a second message scheme for the message type from a plurality of message schemes stored by the one or more storage devices. Additionally, the system 100 is configured to generate a second device driver based on the first device driver and the second message scheme.

By generating a driver for a medical device by starting with a first driver (e.g. a generic driver) and testing the communication with the device, a new or updated driver for the medical device can be generated at low effort compared with conventional methods of developing drivers. The turn-around time and/or efficiency for the development of drivers for medical devices may be improved.

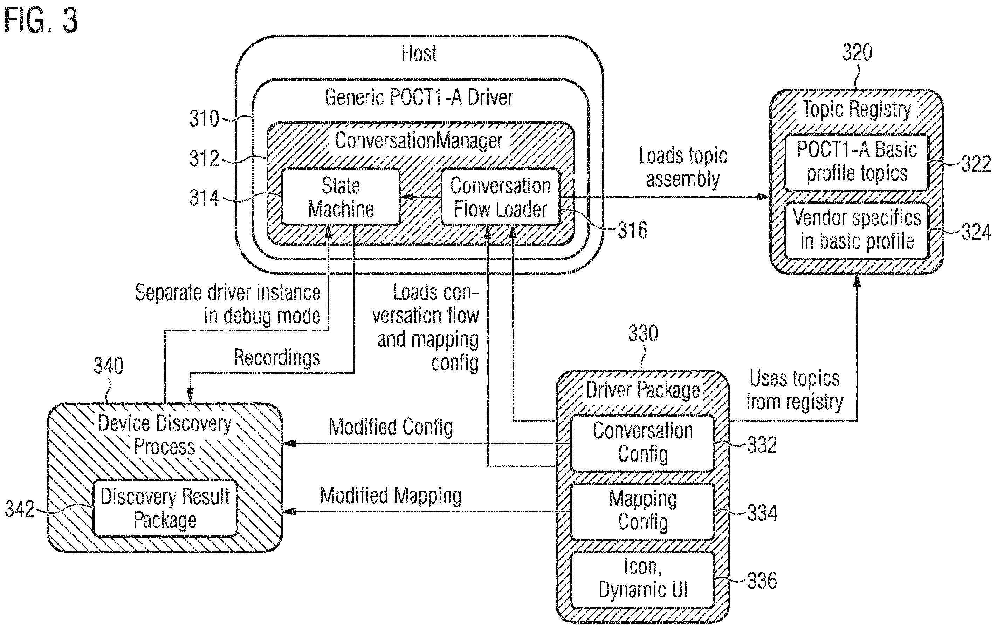

The communication request 102 may be a first message to start a communication with, for example, a message sent from a medical device to a system 100 to which the medical device is connected. For example, if the medical device works according to the POCT01-A2 standard, the medical device may send a hello message (e.g. HEL.R01) to the middleware (e.g. observation reviewer of POCT01-A2 standard) or host of driver framework (e.g. as shown in Fig. 3) executed by the system 100. The communication request 102 may contain information for the system 100 to determine if a conversation should occur and, if so, how it should take place (e.g. using what connection profile, concerning what topics, and/or involving what directives). Alternatively, a communication request may have been sent to the medical device (e.g. by a middleware) or to a middleware of the medical device and the medical device or the middleware of the medical device sends a response to the communication request. The response to the communication request may contain information for the system 100 how it should take place (e.g. using what connection profile, concerning what topics, and/or involving what directives).

The communication request 102 may be received through an input and/or output interface of the system 100 connected (e.g. by a wired and/or wireless connection) to the medical device.

The system 100 transmits the message 106 of the message type to the medical device in direct response to the communication request 102, after an initial communication or during a communication with the medical device.

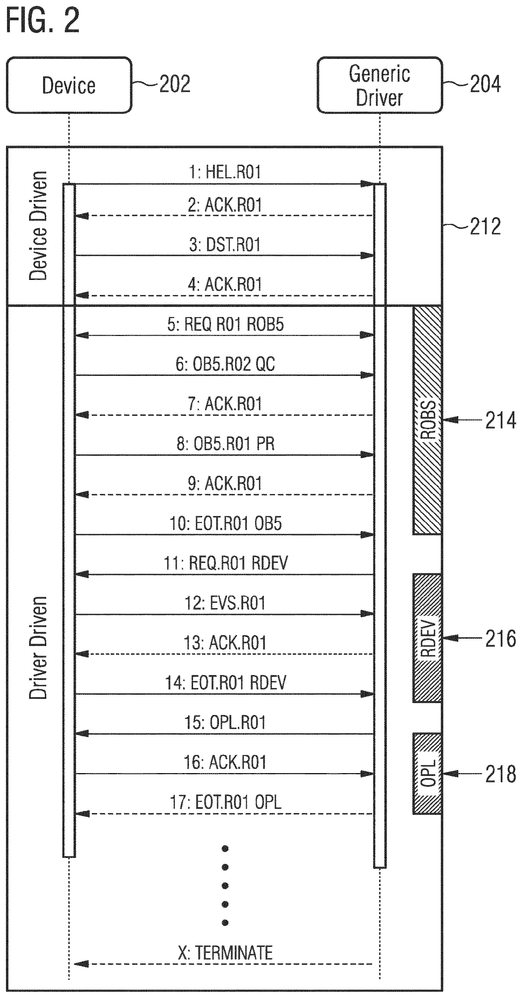

A message type may relate to a specific category or type of information that a medical device can exchange with the system 100 (e.g. a middleware) during communication. The message types may be predefined subjects or areas of data that the device and the system 100 may utilize during their interaction. Each message type may correspond to a particular kind of data that the device needs to send, receive, and/or request from the system 100. For example, a message type may be a topic of the POCT1-A or POCT01-A2 standard (e.g. ROBS, RDEV or OPL as shown in Fig. 2).

A message scheme for the message type may be a predefined sequence of messages which is transmitted between the system 100 and the medical device to exchange the information of the message type. For example, the message scheme may be a sequence of messages of a topic of the POCT1-A or POCT01-A2 standard. For example, a sequence of messages of a topic "observations" may be a first message "request observations" (e.g. REQ.R01 ROBS in Fig. 2) from the system to the medical device, a second message "observations" (e.g. OBS.R02 QC in Fig. 2) from the medical device to the system, and a third message "acknowledgment" (e.g. ACK.R01 in Fig. 2) from the system to the medical device. The first message scheme for the message type may be a first predefined sequence or a default sequence of messages, may comprise a first definition or a default definition of at least one message of the message type, and/or may comprise a first mapping of one or more device parameters (e.g. last name) to one or more system parameters (e.g. surname). For example, a definition of a message may define fields of the message and an order of fields.

A message of a message type may be one of the messages or the message defined in the message scheme for the message type. For example, a message of a topic of the POCT1-A or POCT01-A2 standard may be a message from the sequence of messages defined by the message scheme for the topic. For example, the message of the message type may be a transmission request (e.g. of a request for a transmission of observations) and the system 100 may be configured to receive at least one of an acknowledgement or observation (e.g. quality control information or patient record information) in response to the transmission request. For example, for the topic "observations", a message of the message type may be "request observations" (e.g. REQ.R01 ROBS in Fig. 2), "observations" (e.g. OBS.R02 QC in Fig. 2) or "acknowledgment" (e.g. ACK.R01 in Fig. 2).

The message of the message type may be transmitted through an input and/or output interface of the system 100 connected (e.g. by a wired and/or wireless connection) to the medical device.

The first device driver may be a generic driver for medical devices. For example, the first device driver may be a driver for exchanging messages of a plurality of predefined message types between the system 100 (e.g. a middleware executed by the system) and a medical device.

For example, a device driver is a software component responsible for managing communication between a medical device and a middleware system. A device driver for medical devices may be a specialized software component that allows the system 100 or a software executed by the system (e.g. middleware or operating system or a medical information system) to communicate with a medical device. The device driver may enable communication between the system and the medical device so that information and instructions may be communicated and configurations may be made to the medical device. For example, device drivers serve as the intermediary between the medical device and the higher-level applications or systems that need to interact with it, such as the middleware, a clinical information system, a laboratory information system, or a point-of-care testing system. A generic device driver may have default or standardized driver capability (e.g., POCT1-A or POCT01-A2 capability), incorporating standard topics. The middleware may be a POC data manager and/or observation reviewer unit of the POCT01-A2 standard.

The medical device may send an error message if an unknown message, a message of an unknown message type, a message of a message type according to an unknown message scheme or a message with an unknown field is received. The error message may be a message indicative of an inaccuracy or a deviation from what was expected or intended in the communication. The error message may indicate only in general that an error occurred or may contain information on the error. For example, the error message may be an error acknowledgement message or escape message of the POCT01-A2 standard.

For example, after receiving the error message (e.g. in reaction to the transmission of the message), the system 100 is configured to select a second message scheme for the message type. The system 100 may be configured to select a second message scheme for the message type based on information contained by the error message (e.g. error details) or based on device information (e.g. compatibility to standard or vendor information) or may select the next message scheme for the message type contained by the plurality of message schemes. The second message scheme for the message type may differ from the first message scheme by the order of messages in the sequence of messages, by at least one field of at least one message of the message type, by a message added or removed from the first message scheme, and/or by a different mapping of a device parameter to a system parameter.

The plurality of message schemes may comprise different possible message schemes for a plurality of message types. For example, the plurality of message schemes may comprise two or more different message schemes for a first message type and two or more different message schemes for a second message type. The plurality of message schemes may comprise a plurality of message schemes for each of a plurality of message types. The plurality of message schemes may be stored in a data base or a file. The plurality of message schemes may be or may represent a topic registry comprising message schemes for topics of the POCT1-A or POCT01-A2 or another communication standard and/or vendor specific message schemes. A topic registry may be a repository of individual topics accessible to the driver, e.g., during boot-up via a configuration file. A topic may be a building block (e.g. smallest building block) of the topic registry, containing logic for its domain. New message schemes may be added to the plurality of message schemes if a new standard for medical devices is issued or if a vendor defines a new message scheme for a medical device.

The second driver may be generated by replacing a driver portion relating to the first message scheme by a driver portion relating to the second message scheme. For example, the first device driver may comprise a conversation configuration file containing a list of message types and corresponding message schemes for the communication with a medical device. A message scheme of a message type may be replaced in the conversation configuration file by the second message scheme to generate the second driver. The second driver may be generated by the one or more processors 110. For example, a conversation configuration file for a device driver may be a file read during driver boot-up, containing settings and mappings. A mapping configuration may be a configuration enabling the mapping of data fields from a medical device to a middleware, allowing customization. The second device driver may be a final device driver for communicating with the medical device during normal operation. The system 100 may generate one or more intermediate device drivers until the system 100 generates the second driver that can be used for normal operation of the medical device.

The first device driver and the second device driver may comprise a state machine and a conversation flow loader. The conversation flow loader may load message schemes from the plurality of message schemes depending on the state of the state machine. For example, the second device driver may comprise a conversation flow loader configured to load the second message scheme from the plurality of message schemes.

The system 100 may provide the second device driver for usage (e.g. by a middleware) or may use the second device driver for communicating with the medical device. For example, the system 100 may run a middleware, which generates the second device driver and uses the second device driver for the communication with the medical device during normal operation. For example, the system 100 may be configured to transmit a message of the message type to the medical device according to the second message scheme for the message type using the second device driver.

The system 100 may be able to generate different device drivers for various medical devices. For example, the system 100 may be configured to receive a communication request or a response to a communication request from a second medical device and transmit a message of a second message type to the second medical device according to a first message scheme for the second message type using the first device driver. The first device driver may be a generic driver used for all initial communications with a new, unknow or modified device (e.g. after a firmware update) or may be a generic driver for a group of medical devices (e.g. from the same vendor, compatible with the same communication standard and/or having the same functionality). The second message type may be the same message type as for the first medical device, but for different devices errors may occur for different message types so that the second message type may also differ from the first message type.

The system 100 may be configured to receive an error message from the second medical device in response to the message of the second message type and select a second message scheme for the second message type from the plurality of message schemes stored by the one or more storage devices 120. Additionally, the system 100 may be configured to generate a third device driver based on the first device driver and the second message scheme for the second message type. The system 100 may be configured to generate individual device drivers for a plurality of different medical devices based on the first device driver. By using the same device driver for different medical devices as a starting point, an efficient process may be enabled to generate individual device drivers for different medical devices.

The system 100 may provide the third device driver for usage or may be configured to transmit a message of the second message type to the second medical device according to the second message scheme for the second message type using the third device driver.

The system 100 may be configured to detect a compatibility of the medical device with a communication standard based on device information (e.g. contained by the communication request or by another message received from the medical device). The system 100 may be configured to select a second message scheme from the plurality of message schemes, which is compatible with the communication standard. Additionally, the system 100 may be configured to select the first device driver from a plurality of generic drivers, which is compatible with the communication standard.

The system 100 may be configured to modify a message scheme of the plurality of message schemes based on information on a mapping of a device parameter to a system parameter (e.g. a parameter of the middleware or a laboratory or healthcare information system). For example, a medical device may be configured to use a device parameter called "last name", while the system parameter may be called "surname". The system 100 may modify an existing message scheme based on a new or modified mapping of a device parameter to a system parameter to generate a new message scheme for a message type. The new message scheme for the message type may be added to the plurality of messages schemes.

The medical device may be Point-of-Care Testing (POCT) or another medical device. The medical device may be a device compatible with the POCT1-A or POCT01-A2 standard. The medical device may be a hand-held device, for example, a portable diagnostic device that is used directly at the patient's side. Examples could be glucose meters used by patients with diabetes or other similar devices designed for quick diagnostic results. The medical device may be any home care monitoring devices, for example for blood pressure or for transcutaneous measurements of, e.g., partial pressures of oxygen (pO2) and/or carbon dioxide (pCO2) and pulse oximetry measurements. The medical device may be a bench-top analyzers, for example, a small laboratory device that can be placed on a bench or tabletop and is often used in settings such as remote or satellite laboratories close to the patient. They may be designed to conduct more complex tests that are still accessible outside of a central lab. The medical device may be an integrated module in another instrumentation, for example, a diagnostic test module that is integrated into other medical instruments like patient monitors. For example, the medical device may be a blood gas analyzer integrated into a bedside patient monitor or an analyzer apparatus configured to analyze biological samples used in the field of clinical analysis where clinical personnel may acquire test results and/or measurement results from analyzer apparatuses configured to analyze biological samples, such as samples of bodily fluids.

Some medical devices have built-in networking capabilities that allow them to connect directly to the hospital's local area network or wireless network. This connection enables continuous communication with the system 100 (e.g. Observation Reviewer) without the need for user intervention. In many hospital settings, devices may communicate with the system 100 via an access point. The access point acts as an intermediary, consolidating data from one or more devices and forwarding it to the system 100 over the network. Medical devices that do not have direct network capabilities can connect to an access point using an infrared (IrDA) link or a wired connection. The access point then relays the information to the system 100 using network protocols. Some handheld or portable medical devices require periodic docking to upload data. The docking station provides both a physical connection and, in some cases, power to the device. Once docked, the device can initiate data transfer to the system 100.

The POCT01-A2 standard (Point-of-Care Testing 1-A2 Standard) is a communication protocol for interoperability between point-of-care testing devices and laboratory or healthcare information systems. A laboratory information system may be a software system that records, manages, and stores data for clinical laboratories.

More details and aspects are mentioned in connection with the examples described above or below. The example shown in Fig. 1 may comprise one or more optional additional features corresponding to one or more aspects mentioned in connection with the pro-posed concept or one or more examples described above or below (e.g. Fig. 2-9).

Fig. 2 shows an example of a conversation flow between a medical device 202 and a host (e.g. middleware or laboratory or healthcare information system) or a system for generating a device driver for a medical device using a generic driver 204. In this example, the medical device 202 is a device compatible to the POCT01-A2 standard. The medical device sends a HEL.R01 message representing a communication request. The HEL.R01 message is part of the topic "Device Status" 212 that is driven or started by the medical device 202. The host or system may use a generic driver 204 (e.g. first device driver) to initiate the topics ROBS 214, RDEV 216 and OPL 218. ROBS 214, RDEV 216 and OPL 218 are examples of conversation building blocks taken from the topic registry which may track the topic implementations by device manufacturer, device type and/or device software version. For example, the driver driven part of the conversation flow is not hardcoded but based on a conversation flow configuration file.

In any of these topics an error may occur, if the generic driver 204 is not suitable for the medical device 202. In such a case, the medical device 202 sends an error message and the host or system may adapt the generic driver 204 to generate a suitable driver (e.g. second device driver) as described above or below. Fig. 2 illustrates an example of topics loaded from the topic registry.

More details and aspects are mentioned in connection with the examples described above or below. The example shown in Fig. 2 may comprise one or more optional additional features corresponding to one or more aspects mentioned in connection with the pro-posed concept or one or more examples described above (e.g. Fig. 1) or below (e.g. Fig. 3-9).

Fig. 3 shows a schematic illustration of a system for generating a device driver. The system comprises a host comprising a generic POCT1-A or POCT01-A2 driver 310. The driver 310 comprises a conversation manager 312 including a state machine 314 and a conversation flow loader 316. The state machine 314 gets the state from the different topics so that the state machine 314 knows where in the conversation flow it is. A topic might include exchanging several messages between the device and the driver, during the execution course of a topic, the messages will have a logical sequence, for instance a request will be followed by an acknowledgement, or one segment of data message is followed by another segment of a data message, or a status message is followed by an action request. A state machine, which stores where is it now and decides where the next step should be, might be used to orchestrate the logical sequence of messages, or in another word, control the conversation flow. For the observations topic, the device might have several observations to send to the driver. The topic may then persist all previously received segments of observation in the state machine until all segments have been received and then process the observations. The conversation flow loader 316 is an aggregation of configurations packaged. The conversation flow loader 316 is provided and/or installed together with the Generic POCT1-A or POCT01-A2 driver 310.

The conversation flow loader 316 loads topic assemblies from the topic registry 320. The topic registry 320 comprises POCT1-A or POCT01-A2 basic profile topics 322 and vendor specifics in basic profiles 324.

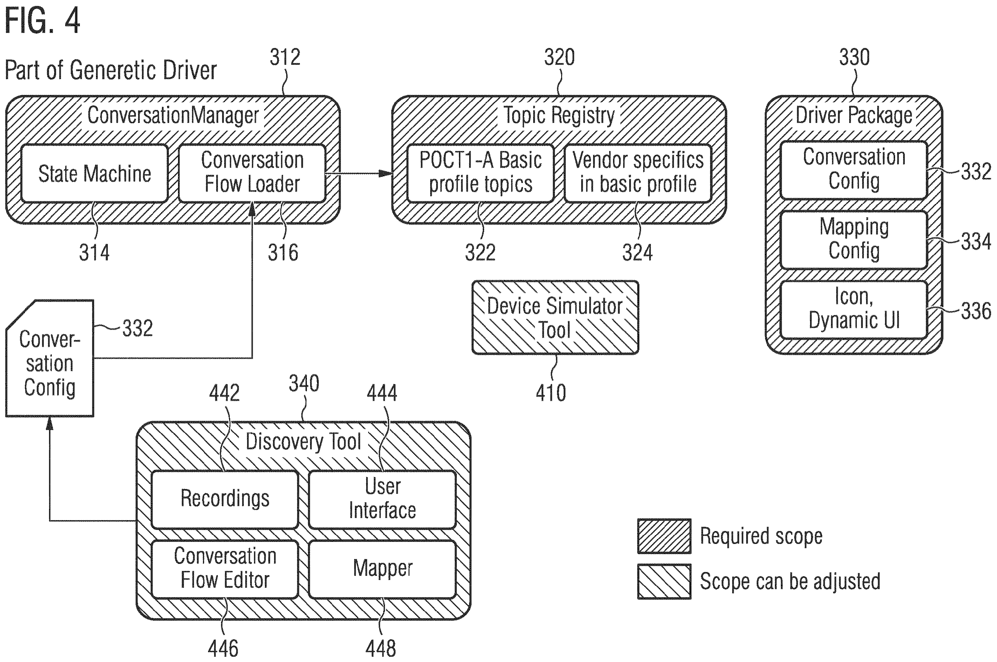

A driver package 330 may comprise a conversation configuration file 332, a mapping configuration file 334 and an icon and/or dynamic user interface file 336. The driver package 330 may contain an icon of the device that can be provided by the system 100 for displaying by a user interface or may contain a data structure that describes a device specific user interface (e.g. for configuring the device) that can be processed by the system 100. The driver package 330 may be an aggregation of configurations packaged. It may be provided and installed together with the generic POCT1-A or POCT01-A2 driver 310. The conversation configuration file 332 may be created based on a sequence of topics given by a conversation flow editor (e.g. Fig. 4), and may comprise a default sequence of topics or an adapted list of topics generated during the generation of a driver for a specific medical device. The mapping configuration file 334 may include information on a mapping of device specific parameters to system specific parameters (e.g. parameters of the middleware). Device specific topics may be updated or added through the driver package. In case none of the basic profile topics 322 or vendor specific topics in the basic profiles 324 in the registry can fulfill what needs to be done, vendor specific topics can be added and/or updated by driver packages into the topic registry 320.

The conversation flow loader 316 of the generic driver 310 loads the conversation configuration file 332 and the mapping configuration file 334 from the driver package 330. The conversation configuration file 332 uses the topics from the registry 320. The driver package 330 references the topic registry 320 where the conversation flow loader 316 loads the topic with the reference from the driver package 330.

Additionally, a device discovery process or tool 340 may be implemented comprising a discovery result package 342. The discovery process 340 may produce a discovery result package 345 that may provide inputs to the driver package conversation configuration file 332 and/or mapping configuration file 334. The results are created with discovery tools. The device discovery process 340 receives recordings (e.g. of communications between system/host and medical device) from the generic driver 310, a modified configuration from the conversation configuration file 332 and/or a modified mapping from the mapping configuration file 334. The discovery process 340 may have a dedicated driver running in debug mode and may capture the communication. Further, it may modify the conversion configuration 332 and/or the mapping configuration 334 during the discovery process 340. The device discovery process 340 may run a separate driver instance in a debug mode (e.g. as a communication counterpart) to discover the device communication behavior.

Fig. 3 illustrates the components of an example of a driver framework. An aspect may be to optimize the reuse of standard topics whenever possible. In case of a failure with the newly introduced driver, and if mapping cannot resolve the issue, modifications to the topics may be made. These specific topics may be enhanced with additional identifiers in the topic registry 320 to match them with the required driver functionality for a specific device type or model. The smallest building block may be a topic, containing the logic for its domain. If additional functionality or logic is required, it may be incorporated directly into the topic. Each topic may be developed as an independent assembly, serving as a conversation building block of the solution. This approach may enable individual topics to assume specific responsibilities and may introduce capabilities for independent topic management, including enabling/disabling, upgrading/downgrading, and separate testing/validation.

The conversation manager 312 may be a component responsible for configuring conversation flow between the device driver and medical devices, facilitating seamless communication and data exchange.

More details and aspects are mentioned in connection with the examples described above or below. The example shown in Fig. 3 may comprise one or more optional additional features corresponding to one or more aspects mentioned in connection with the pro-posed concept or one or more examples described above (e.g. Fig. 1-2) or below (e.g. Fig. 4-9).

Fig. 4 shows a schematic illustration of a system for generating a device driver. The conversation manager 312, the topic registry 320 and the driver package 330 may be implemented as described in connection with Fig. 3. The conversation configuration file 332 of the driver package 330 may be generated or modified by a discovery tool. The discovery tool 340 may comprise recordings 442, a user interface 444, a conversation flow editor 446 and a mapper 448. The user interface 444 may enable a technician (e.g. a field service engineer who is on the field at POC and is responsible for upgrading, servicing and troubleshooting devices) to adjust the conversation flow and topics enabled as well as manage the playback of the recordings. The recordings 442 may be captured as feedback from the device during the device discovery process. The conversation flow editor 446 may enable an easy manipulation of the conversation flow configuration. The mapper 448 may enable an easy manipulation of mapping configuration.

Additionally, a device simulator tool 410 may be implemented. The device simulator tool 410 may use the POCT1-A or POCT01-A2 protocol as a base scheme to establish a conversation with a device and then record it. Through the conversation flow editor 446 the conversation can be modified to manage the devices behavior that does not follow the POCT1-A or POCT01-A2 standard.

A device discovery unit and/or debug tool may comprise a generic driver component for creating a configurable conversation flow and for pulling topics to be used for the conversation flow, a search component for establishing an initial conversation flow (e.g. best-match search for initial conversation flow), and a parameter mapping component for mapping the POCT1-A or POCT01-A2 protocol with the middleware. Other protocols than POCT1-A or POCT01-A2 can be used for the conversation flows depending on what protocol is supported by the POCT devices.

Fig. 4 illustrates an example of a general configurable driver framework comprising a conversation manager 312, a topic registry 320, and driver package 330. The conversation manager 312 comprises a state machine 314 and a conversation flow loader 316 and pulls topics with meta data for the conversation flow from the topic registry 320 to create the conversation flow, for example. In addition to the framework, a device simulator tool 410 and a device discovery tool 340 are illustrated. The discovery tool 340 may provide a conversation configuration based on the capabilities of the POCT device that has been discovered by the discovery tool 340. The device simulator tool 410 may simulate a conversation flow between the middleware and the POCT device in order to test and verify the conversation flow, which conversation flow also can be described as a communication protocol.

Different scenarios may be encountered on the field when discovering a POCT device. For example, when a POCT devices needs new firmware, there is a high likelihood that the existing building blocks are the same as in the previous version of the firmware meaning that the driver workflow will update the POCT device with the needful and enable on-site verification. Another example is when a new POCT device from a known vendor is discovered, there is a high likelihood that the existing topics are the same as for other POCT devices from the same vendor meaning that the driver workflow will update the POCT device with the needful and enable on-site implementation. Yet another example is when a new POCT device from an unknown vendor is discovered, there is a high likelihood that the standard POCT1-A or POCT01-A2 topics (building blocks) are the same as for other POCT devices meaning that the driver workflow will update the POCT device with the needful and enable on-site implementation. Another example is when none of the other scenarios apply, and when the IT specialist creates a connection request for this particular discovered POCT device, wherein the IT specialist may gather information about the building blocks used and the parameters that need manipulation for the development team in order for them to add/modify new topics in the topics registry or to modify the configuration flow sequence to enable the automatic process of configuring drivers at the customer site.

A feasibility study (e.g. document review of vendor specification) and/or technical investigation (e.g. validating real device with vendor specific documentation) process on the field may identify a device with new firmware with a high chance that existing building blocks will work. An on-site verification and a notification to release the process may be implemented. The process may identify a new device from a known vendor with high chance that vendor specific topics use the same implementation. For a new device from an unknown vendor, standard POCT01-A2 topics may be tested.

A feasibility study FS and/or technical investigation Tl package may include used building blocks, recordings and manipulated parameters. As input for development, a Tl report may be created out of the package which contains all the recordings gathered during the test and information about building blocks used during the discovery process. The development team may determine if there are any required topics that are missing in the registry and estimate the effort for implementing them. New implementations can be tested on the field using the discovery tool before official release.

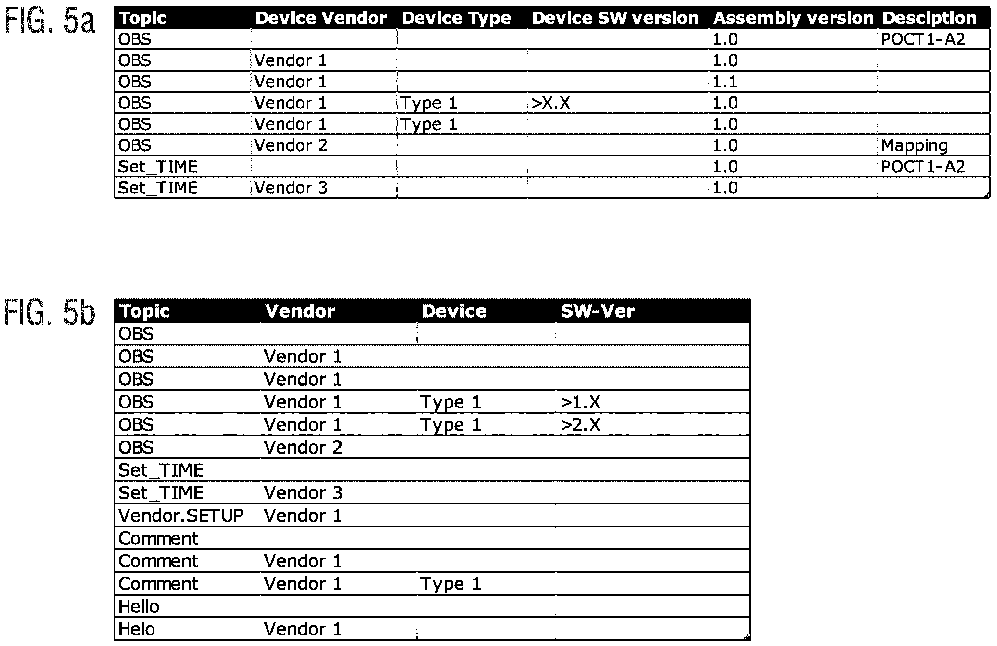

More details and aspects are mentioned in connection with the examples described above or below. The example shown in Fig. 4 may comprise one or more optional additional features corresponding to one or more aspects mentioned in connection with the pro-posed concept or one or more examples described above (e.g. Fig. 1-3) or below (e.g. Fig. 5a-9).

Fig. 5a illustrates an example of a part of a topic registry which acts as a repository of individual topics accessible to the driver during boot-up via a driver package configuration. Initially, standard topics may be supported in the topic registry, with modified or newly introduced topics added over time as necessary. A registry of implemented topics (e.g. representing building blocks) with metadata is shown. Examples associated with building blocks, which are reusable and can be pulled-in for a specific driver, are shown. The device vendor may be a manufacturer or provider of medical devices. The versioning may be a process of assigning unique identifiers to different versions of topics, enabling management of updates and bug fixes.

Fig. 5b illustrates an example of a part of a topic registry comprising topics with metadata which is a repository of building blocks that can be pulled-in when configuring specific conversation flows for specific drivers.

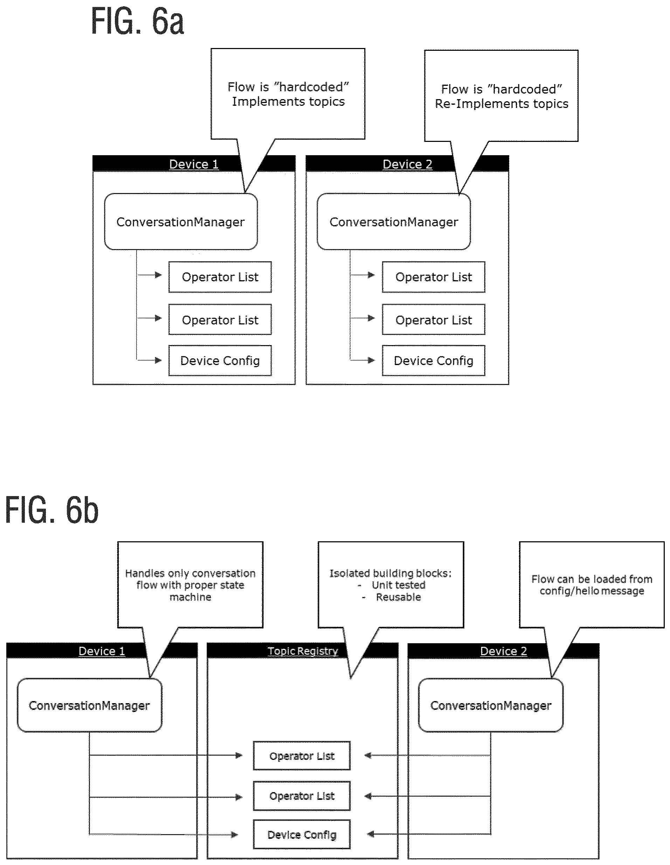

More details and aspects are mentioned in connection with the examples described above or below. The example shown in Fig. 5a and 5b may comprise one or more optional additional features corresponding to one or more aspects mentioned in connection with the pro-posed concept or one or more examples described above (e.g. Fig. 1-4) or below (e.g. Fig. 6a-9).

Fig. 6a shows a schematic illustration of hardcoded drivers. Figure 6a illustrates hardcoded conversation flows with duplicated code (e.g. missing state machines) generating complexity in drivers and being hard to maintain compared to the driver framework as illustrated in Figure 3, for example.

Fig. 6b shows a schematic illustration of drivers using a topic registry. Figure 6a illustrates an example of conversation flows according to the driver framework as illustrated in Figure 3, for example. The conversation flows are handled by conversation managers with state machines by means of topics with metadata (e.g. building blocks) obtained from the topic registry. The topic registry may contain isolated building blocks, which are unit tested and reuseable. The flow can be loaded from the config/hello message. For example, the hello message may contain a list of topics supported by the device and a flow can be derived from the list of supported topics. The topic registry may comprise message schemes for operator list, patient, list and device configuration, for example.

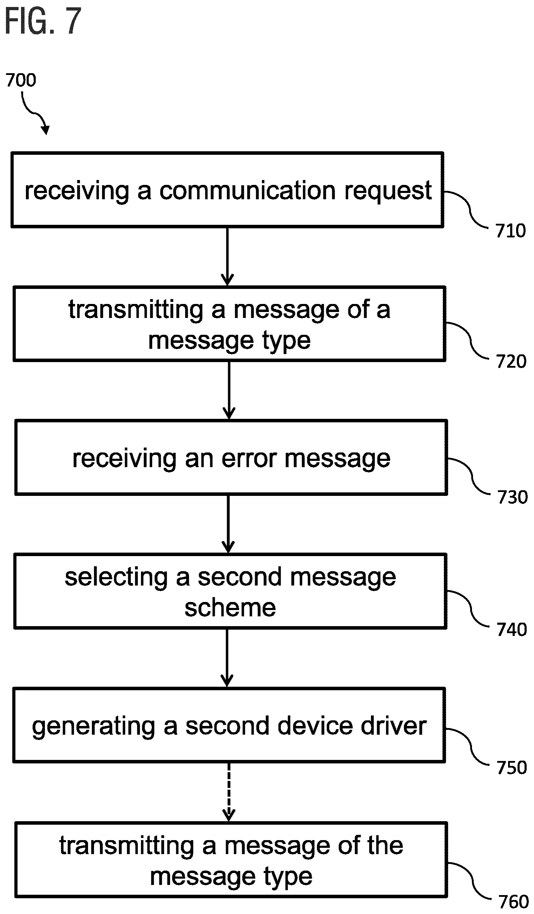

More details and aspects are mentioned in connection with the examples described above or below. The example shown in Fig. 6a or 6b may comprise one or more optional additional features corresponding to one or more aspects mentioned in connection with the pro-posed concept or one or more examples described above (e.g. Fig. 1-5b) or below (e.g. Fig. 7-9).

Fig. 7 shows a computer-implemented method 700 for generating a device driver for a medical device. The method 700 comprises receiving 710 a communication request or a response to a communication request from a medical device and transmitting 720 a message of a message type to the medical device according to a first message scheme for the message type using a first device driver. Further, the method 700 comprises receiving 730 an error message from the medical device in response to the message and selecting 740 a second message scheme for the message type from a plurality of message schemes. Additionally, the method 700 comprises generating 750 a second device driver based on the first device driver and the second message scheme. The method may comprise transmitting 760 a message of the message type to the medical device according to the second message scheme for the message type using the second device driver.

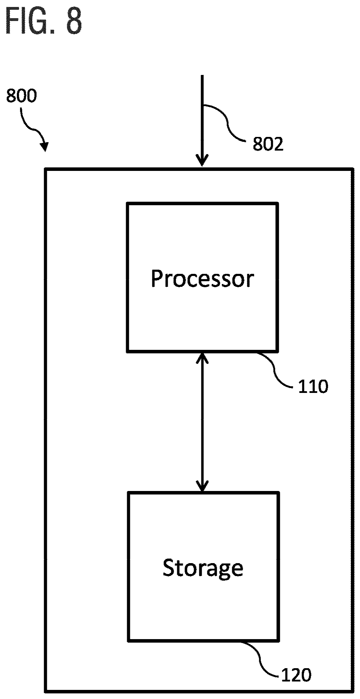

More details and aspects are mentioned in connection with the examples described above or below. The example shown in Fig. 7 may comprise one or more optional additional features corresponding to one or more aspects mentioned in connection with the pro-posed concept or one or more examples described above (e.g. Fig. 1-6) or below (e.g. Fig. 8-9).

Fig. 8 shows a system 800 for generating a device driver. The system 800 comprises one or more processors 110 and one or more storage devices 120. The system 800 is configured to obtain device information 802 of a medical device and provide a message of a message type to a device simulator according to a first message scheme for the message type using a first device driver. Further, the system 800 is configured to simulate, by the device simulator based on the device information, a first response of the medical device to the message transmitted according to the first message scheme. Additionally, the system 800 is configured to select a second message scheme for the message type from a plurality of message schemes stored by the one or more storage devices 120, if the first response indicates an error. Furthermore, the system 800 is configured to generate a second device driver based on the first device driver and the second message scheme and provide a message of the message type to the device simulator according to the second message scheme for the message type using the second device driver. Additionally, the system 800 is configured to simulate, by the device simulator based on the device information, a second response of the medical device to the message transmitted according to the second message scheme.

By generating a driver for a medical device by starting with a first driver (e.g. generic driver) and simulating the communication with the device, a new or updated driver for the medical device can be generated at low effort compared with conventional methods of developing drivers. The turn-around time and/or efficiency for development of drivers for medical devices may thus be improved.

The device information 802 may comprise information on a compatibility to a communication standard (e.g. POCT1-A or POCT01-A2 standard), vendor information, information on device capabilities and/or information on supported message types. The device information 802 may comprise sufficient information for the device simulator to simulate a communication behavior of the medical device. The device information may be received from a medical device being connected to the system 100 (e.g. contained by a communication request or a device status message), may be received from a device vendor or may be obtained from a database or a device specification. The device information 802 may be received through an input and/or output interface of the system 100.

The system 100 provides the message of the message type to the device simulator in reaction to a communication request from a medical device or after a user request and after obtaining the device information 802. The device simulator may be configured to simulate a device behavior of the medical device based on the device information. The device simulator may be configured to replicate operations, behaviors, and/or conditions related to communication aspects of a medical device. The device simulator may be able to work as a device based on recordings to simulate different devices and different conversations of a device. For example, the device simulator may load a pre-recorded session of communication messages. There can be many pre-recorded sessions that capture different scenarios and/or behaviors of a device. Then, the simulator may be configured to play a session or to simulate a particular device behavior. The device simulator may be implemented as described for the device simulator tool 410 of Fig. 4.

A message type may relate to a specific category or type of information that a medical device can exchange with the system 100 (e.g. a middleware) during communication. The message types may be predefined subjects or areas of data that the device and the system 100 discuss during their interaction. Each message type may correspond to a particular kind of data that the device needs to send, receive, and/or request from the system 100. For example, a message type may be a topic of the POCT1-A or POCT01-A2 standard (e.g. ROBS, RDEV or OPL as shown in Fig. 2).

A message scheme for the message type may be a predefined sequence of messages which is transmitted between the system 100 and the medical device to exchange the information of the message type. For example, the message scheme may be a sequence of messages of a topic of the POCT1-A or POCT01-A2 standard. For example, a sequence of messages of a topic "observations" may be a first message "request observations" (e.g. REQ.R01 ROBS in Fig. 2) from the system to the medical device, a second message "observations" (e.g. OBS.R02 QC in Fig. 2) from the medical device to the system and a third message "acknowledgment" (e.g. ACK.R01 in Fig. 2) from the system to the medical device. The first message scheme for the message type may be a first predefined sequence or a default sequence of messages, may comprise a first definition or a default definition of at least one message of the message type, and/or may comprise a first mapping of one or more device parameters (e.g. last name) to one or more system parameters (e.g. surname). For example, a definition of a message may define fields of the message and an order of fields.

A message of a message type may be one of the messages or the message defined in the message scheme for the message type. For example, a message of a topic of the POCT1-A or POCT01-A2 standard may be a message from the sequence of messages defined by the message scheme for the topic. For example, the message of the message type may be a transmission request of a request for a transmission of observations and the system 100 may be configured to receive at least one of quality control QC information or patient record PR information in response to the transmission request. For example, for the topic "observations", a message of the message type may be "request observations" (e.g. REQ.R01 ROBS in Fig. 2), "observations" (e.g. OBS.R02 QC in Fig. 2) or "acknowledgment" (e.g. ACK.R01 in Fig. 2).

The first device driver may be a generic driver for medical devices. For example, the first device driver may be a driver for exchanging messages of a plurality of predefined message types between the system 100 (e.g. a middleware executed by the system) and a medical device.

For example, a device driver is a software component responsible for managing communication between a medical device and a middleware system. A device driver for medical devices may be a specialized software component that allows the system 100 or a software executed by the system (e.g. middleware or operating system or a medical information system) to communicate with and/or control the hardware of a medical device. For example, device drivers serve as the intermediary between the medical device and the higher-level applications or systems that need to interact with it, such as the middleware, a clinical information system, a laboratory information system, or a point-of-care testing system. A generic device driver may have default or standardized driver capability (e.g., POCT1-A or POCT01-A2 capability), incorporating standard topics. The middleware may be a POC data manager and/or observation reviewer unit of the POCT01-A2 standard.

The device simulator may send the first response (e.g. an error message) indicating an error if an unknown message, a message of an unknown message type, a message of a message type according to an unknown message scheme or a message with an unknown field was provided to the device simulator. The first response may indicate only in general that an error occurred or may contain information on the error. For example, the first response may be an error acknowledgement message or escape message of the POCT01-A2 standard.

For example, after receiving the first response (e.g. in reaction to the transmission of the message), the system 100 is configured to select a second message scheme for the message type. The system 100 may be configured to select a second message scheme for the message type based on information contained by the first response (e.g. error details) and/or based on device information (e.g. compatibility to standard or vendor information) or may select the next message scheme for the message type contained by the plurality of message schemes. The second message scheme for the message type may differ from the first message scheme by the order of messages in the sequence of messages, by at least one field of at least one message of the message type, by a message added or removed from the first message scheme, and/or by a different mapping of a device parameter to a system parameter.

The plurality of message schemes may comprise different possible message schemes for a plurality of message types. For example, the plurality of message schemes may comprise two or more different message schemes for a first message type and two or more different message schemes for a second message type. The plurality of message schemes may comprise a plurality of message schemes for each of a plurality of message types. The plurality of message schemes may be stored in a database or a file. The plurality of message schemes may be or may represent a topic registry comprising message schemes for topics of the POCT1-A or POCT01-A2 or another communication standard and/or vendor specific message schemes. A topic registry may be a repository of individual topics accessible to the driver during boot-up via a configuration file. A topic may be a building block (e.g. smallest building block) of the topic registry, containing logic for its domain. New message schemes may be added to the plurality of message schemes if a new standard for medical devices is issued or if a vendor defines a new message scheme for a medical device.

The second driver may be generated by replacing a driver portion relating to the first message scheme by a driver portion relating to the second message scheme. For example, the first device driver may comprise a conversation configuration file containing a list of message types and corresponding message schemes for the communication with a medical device. A message scheme of a message type may be replaced in the conversation configuration file by the second message scheme to generate the second driver. The second driver may be generated by the one or more processors 110. For example, a conversation configuration file for a device driver may be a file read during driver boot-up, containing settings and mappings. A mapping configuration may be a configuration enabling the mapping of data fields from a medical device to a middleware, allowing customization. The second device driver may be a final device driver for communicating with the medical device during normal operation. The system 100 may generate one or more intermediate device drivers until the system 100 generates the second driver that can be used for normal operation of the medical device.

The first device driver and the second device driver may comprise a state machine and a conversation flow loader. The conversation flow loader may load message schemes from the plurality of message schemes depending on the state of the state machine. For example, the second device driver may comprise a conversation flow loader configured to load the second message scheme from the plurality of message schemes.

The device simulator may send the second response indicating successful transmission of the message. A successful transmission may be indicated if the response is an expected to the message of the message type or an acknowledgement of successful reception of the message of the message type. For example, the second response may be an acknowledgement message or an expected response message of the POCT01-A2 standard.

The system 100 may provide the second device driver for usage (e.g. by a middleware) or may use the second device driver for communicating with the medical device. For example, the system 100 may run a middleware, which generates the second device driver and uses the second device driver for the communication with the medical device during normal operation. The system 800 may be configured to transmit a message of the message type to the medical device using the second device driver (e.g. if the second response indicates a successful message exchange). The message of the message type may be transmitted through an input and/or output interface of the system 100 connected (e.g. by a wired and/or wireless connection) to the medical device.

The system 100 may be able to generate different device drivers for various medical devices. For example, the system 100 may be configured to obtain device information of a second medical device and transmit a message of a second message type to the device simulator according to a first message scheme for the second message type using the first device driver. The first device driver may be a generic driver used for all initial communications with a new, unknow or modified device (e.g. after a firmware update) or may be a generic driver for a group of medical devices (e.g. from the same vendor, compatible with the same communication standard and/or having the same functionality). The second message type may be the same message type as for the first medical device, but for different devices errors may occur for different message types so that the second message type may also differ from the first message type.

The system 100 may be configured to simulate, by the device simulator based on the device information from the second medical device, a first response of the second medical device to the message transmitted according to the first message scheme for the second message type. The system 100 may be configured to select a second message scheme for the second message type from the plurality of message schemes stored by the one or more storage devices, if the first response simulated for the second medical device indicates an error. Additionally, the system 100 may be configured to generate a third device driver based on the first device driver and the second message scheme for the second message type. The system 110 may be configured to generate individual device drivers for a plurality of different medical devices based on the first device driver. By using the same device driver for different medical devices as a starting point, an efficient process may be enabled to generate individual device drivers for different medical devices.

The system 100 may be configured to provide a message of the second message type to the device simulator according to the second message scheme for the second message type using the third device driver. Additionally, the system 100 may be configured to simulate, by the device simulator based on the device information, a second response of the second medical device to the message of the second message type transmitted according to the second message scheme for the second message type.

The system 100 may provide the third device driver for usage or may be configured to transmit a message of the second message type to the second medical device according to the second message scheme for the second message type using the third device driver (e.g. if the second response simulated for the second medical device indicates a successful message exchange).

The system 100 may be configured to detect a compatibility of the medical device with a communication standard based on the device information. The system 100 may be configured to select a second message scheme from the plurality of message schemes, which is compatible with the communication standard. Additionally, the system 100 may be configured to select the first device driver from a plurality of generic drivers, which is compatible with the communication standard.

The system 100 may be configured to modify a message scheme of the plurality of message schemes based on information on a mapping of a device parameter to a system parameter (e.g. a parameter of the middleware or a laboratory or healthcare information system). For example, a medical device may use a field called "last name", while the system parameter may be called "surname". The system 100 may modify an existing message scheme based on a new or modified mapping of a device parameter to a system parameter to generate a new message scheme for a message type. The new message scheme for the message type may be added to the plurality of messages schemes.

The medical device may be Point-of-Care Testing (POCT) or another medical device. The medical device may be a device compatible with the POCT1-A or POCT01-A2 standard. The medical device may be a hand-held device, for example, a portable diagnostic device that is used directly at the patient's side. Examples could be glucose meters used by patients with diabetes or other similar devices designed for quick diagnostic results. The medical device may be a bench-top analyzers, for example, a small laboratory device that can be placed on a bench or tabletop and is often used in settings such as remote or satellite laboratories close to the patient. They may be designed to conduct more complex tests that are still accessible outside of a central lab. The medical device may be an integrated module in another instrumentation, for example, a diagnostic test module that is integrated into other medical instruments like patient monitors. For example, a blood gas analyzer integrated into a bedside patient monitor.

Some medical devices have built-in networking capabilities that allow them to connect directly to the hospital's local area network or wireless network. This connection enables continuous communication with the system 100 (e.g. Observation Reviewer) without the need for user intervention. In many hospital settings, devices may communicate with the system 100 via an access point. The access point acts as an intermediary, consolidating data from one or more devices and forwarding it to the system 100 over the network. Medical devices that do not have direct network capabilities can connect to an access point using an infrared (IrDA) link or a wired connection. The access point then relays the information to the system 100 using network protocols. Some handheld or portable medical devices require periodic docking to upload data. The docking station provides both a physical connection and, in some cases, power to the device. Once docked, the device can initiate data transfer to the system 100.

The POCT01-A2 standard (Point-of-Care Testing 1-A2 Standard) is a communication protocol for interoperability between point-of-care testing devices and laboratory or healthcare information systems. A laboratory information system may be a software system that records, manages, and stores data for clinical laboratories.

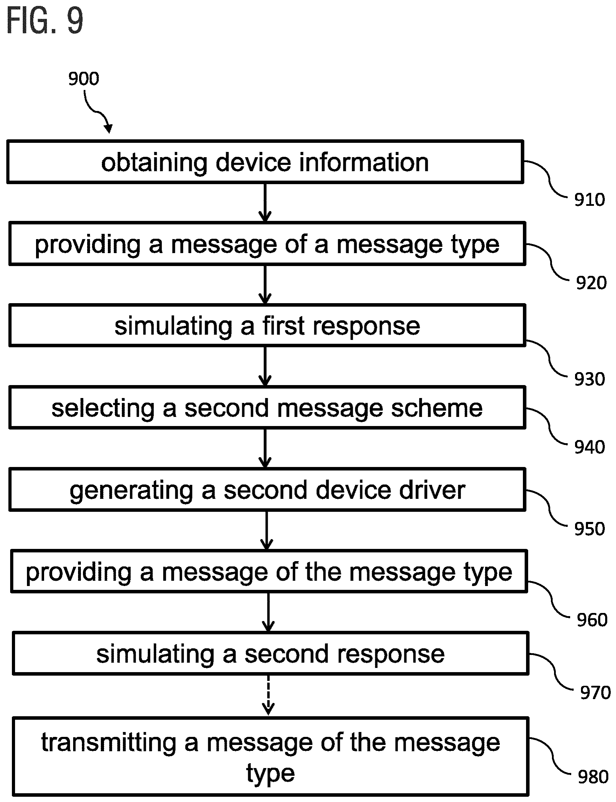

More details and aspects are mentioned in connection with the examples described above or below. The example shown in Fig. 8 may comprise one or more optional additional features corresponding to one or more aspects mentioned in connection with the pro-posed concept or one or more examples described above (e.g. Fig. 1-7) or below (e.g. Fig. 9).

Fig. 9 shows a computer-implemented method 900 for generating a device driver for a medical device. The method 900 comprises receiving 910 device information from a medical device and providing 920 a message of a message type to a device simulator according to a first message scheme for the message type using a first device driver. Further, the method 900 comprises simulating 930, by the device simulator based on the device information, a first response of the medical device to the message transmitted according to the first message scheme. Additionally, the method 900 comprises selecting 940 a second message scheme for the message type from a plurality of message schemes, if the first response indicates an error. Furthermore, the method 900 comprises generating 950 a second device driver based on the first device driver and the second message scheme and providing 960 a message of the message type to the device simulator according to the second message scheme for the message type using the second device driver. Additionally, the method 900 comprises simulating 970, by the device simulator based on the device information, a second response of the medical device to the message transmitted according to the second message scheme. The method 900 may comprise transmitting 980 a message of the message type to the medical device using the second device driver, if the second response indicates a successful message exchange.

More details and aspects are mentioned in connection with the examples described above or below. The example shown in Fig. 9 may comprise one or more optional additional features corresponding to one or more aspects mentioned in connection with the pro-posed concept or one or more examples described above (e.g. Fig. 1-8) or below.

The system 100, 800 may be a local computer device (e.g. a server, a personal computer, a laptop, a tablet computer or a mobile phone) with one or more processors and one or more storage devices or may be a distributed computer system (e.g. a cloud computing system with one or more processors and one or more storage devices distributed at various locations, for example, at a local client and/or one or more remote server farms and/or data centers). In one example, the system 100, 800 may include one or more processors 110 which can be of any type. As used herein, processor may mean any type of computational circuit, such as but not limited to a microprocessor, a microcontroller, central processing unit (CPU), a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA) or any other type of processor or processing circuit. Other types of circuits that may be included in the system 100, 800 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The system 100, 800 may include one or more storage devices 120, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The system 100, 800 may also include a display device, one or more speakers, and a keyboard and/or controller, which can include a mouse, trackball, touch screen, voice-recognition device, or any other device that permits a system user to input information into and receive information from the system 100, 800.

Some examples relate to a configurable driver connectivity framework and/or a configurable driver connectivity workflow. With a general configurable driver framework and/or workflow that is capable of creating instances of drivers configured for specific POCT devices (e.g. automatically on-site when devices are discovered). With this new framework and/or workflow there might not be the need to develop drivers from scratch per POCT device which in turn reduces turn-around time and increases the efficiency of the development process.

According to other concepts, connectivity to POCT devices has been enabled by developing individual interface drivers with limited ability to be modified or changed that enable the POCT devices (e.g., from different manufacturers) to interact with middleware systems. This involves a lot of repetitive work that renders the development process lengthy and cumbersome as even smaller updates require a full development, test and release process. In cases of a driver not working properly the customer have to use emergency procedures until a new driver has been released. Due to this full development process, access to a physical POCT device is required to validate the communication flow between the POCT device and the middleware system and the specification of the given communication protocol. Besides access to the POCT device itself this also entails a significant amount of consumables for quality control and patient test, for example.

Further, operating the POCT device during development requires knowledge and training that would normally not be available within a software development setup. To perform simulated patient tests some POCT devices require the use of real blood to be able to work. This also requires proper training and facility for handling blood samples.

For example, in cases where a POCT device has new firmware that the customer/hospital has not been notified about and which firmware changes the existing driver communication, the customer workflows may be impacted and may require a full development cycle including test and release together with access to the POCT device.

Summarizing, a full development cycle including test and release together with access to the POCT device may be required when a connectivity request for a POCT device is initiated.

By introducing the proposed concept of a general configurable driver workflow, the mentioned difficulties may be addressed by the workflow's ability to configure drivers at the customer (on-site) which for example may remove the need for additional POCT devices for the purpose of testing, accessing and managing blood in a software development setup further shortening the time of delivery of connectivity to POCT devices significantly.

In case a modification of a driver is required then this can in many cases be achieved by mere configuration or mapping within sub-sections of the driver, in so called connectivity components. In cases where modification/development is required then this can be managed with a reduced turn around-time as the connectivity components further comprises individual sub-components.

Some POCT devices only have limited input fields and ability to send information to the middleware system which creates problems for the hospital in terms of following their own procedures and requirements, hence the connectivity components are needed to modify/transform the data that is outputted on the POCT devices to meet these procedures and requirements.

The overall approach of the architecture may involve establishing a framework for a generic POCT01-A2 driver. This framework may incorporate a configuration for managing conversation flow and mapping data fields. Additionally, it may introduce a topic registry containing topics essential for specific conversations, adhering to the POCT01-A2 standard. Other additional standards may also be supported by the architecture.

The proposed workflow may provide an ability to configure drivers at the customer site on demand. An IT specialist may oversee an automatic process where POCT devices are discovered (e.g. by a discovery tool) and wherein the proposed workflow may provide a conversation configuration based on the capabilities of the POCT device that has been discovered by the discovery tool. The IT specialist may also oversee an automatic process where a device simulator tool simulates a conversation flow between the middleware and the POCT device in order to test and verify the conversation flow.

The proposed general configurable driver workflow may enable that: POCT device specific conversation flows can be configured which enable quick turnaround-time for drivers for connecting new or existing POCT devices to middleware services; POCT devices can be discovered on-site (at the customer) reducing test/validation time; parameters and fields for drivers can be mapped/configured with the help of building blocks in the topic registry which provides flexibility and scalability to the driver workflow, for example, by re-using building blocks for several drivers sharing similar conversation flow or by adding new building blocks to the topic registry for specific drivers and their different conversation flows; the release time of new or updated drivers is reduced; the availability of connected devices is increased; easy maintenance is enabled; driver performance is increased; and/or scalability is enabled.

As used herein the term "and/or" includes any and all combinations of one or more of the associated listed items and may be abbreviated as "f".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some or all of the method steps may be executed by (or using) a hardware apparatus, like for example, a processor, a microprocessor, a programmable computer or an electronic circuit. In some examples, some one or more of the most important method steps may be executed by such an apparatus.

Depending on certain implementation requirements, examples of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some examples according to the invention comprise a data carrier having electronically readable control signals, which are capable of cooperating with a programmable computer system, such that one of the methods described herein is performed.

Generally, examples of the present invention can be implemented as a computer program product with a program code, the program code being operative for performing one of the methods when the computer program product runs on a computer. The program code may, for example, be stored on a machine readable carrier.

Other examples comprise the computer program for performing one of the methods described herein, stored on a machine readable carrier.

In other words, an example of the present invention is, therefore, a computer program having a program code for performing one of the methods described herein, when the computer program runs on a computer.

A further example of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further example of the present invention is an apparatus as described herein comprising a processor and the storage medium.

A further example of the invention is, therefore, a data stream or a sequence of signals representing the computer program for performing one of the methods described herein. The data stream or the sequence of signals may, for example, be configured to be transferred via a data communication connection, for example, via the internet.

A further example comprises a processing means, for example, a computer or a programmable logic device, configured to, or adapted to, perform one of the methods described herein.

A further example comprises a computer having installed thereon the computer program for performing one of the methods described herein.

A further example according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some examples, a programmable logic device (for example, a field programmable gate array) may be used to perform some or all the functionalities of the methods described herein. In some examples, a field programmable gate array may cooperate with a microprocessor to perform one of the methods described herein. Generally, the methods are preferably performed by any hardware apparatus.

## Claims

1. A system (100) comprising one or more processors (110) and one or more storage devices (120), wherein the system is configured to:
receive a communication request or a response to a communication request from a medical device;
transmit a message of a message type to the medical device according to a first message scheme for the message type using a first device driver;
receive an error message from the medical device in response to the message;
select a second message scheme for the message type from a plurality of message schemes stored by the one or more storage devices (120); and
generate a second device driver based on the first device driver and the second message scheme.

2. The system of claim 1, wherein the system (100) is configured to generate the second device driver by replacing a driver portion relating to the first message scheme by a driver portion relating to the second message scheme.

3. The system of one of the previous claims, wherein the system (100) is configured to:
receive a communication request or a response to a communication request from a second medical device;
transmit a message of a second message type to the second medical device according to a first message scheme for the second message type using the first device driver;
receive an error message from the second medical device in response to the message of the second message type;
select a second message scheme for the second message type from the plurality of message schemes stored by the one or more storage devices;
generate a third device driver based on the first device driver and the second message scheme for the second message type; and
transmit a message of the second message type to the second medical device according to the second message scheme for the second message type using the third device driver.

4. The system of one of the previous claims, wherein the plurality of message schemes comprises a plurality of message schemes for each of a plurality of message types.

5. The system of one of the previous claims, wherein the plurality of message schemes is stored in a database or in a file.

6. The system of one of the previous claims, wherein the message of the message type is a transmission request, wherein the system is configured to receive at least one of an acknowledgement or observation in response to the transmission request.

7. The system of one of the previous claims, wherein the message type represents a topic of the POCT01-A2 standard or POCT1-A standard.

8. The system of one of the previous claims, wherein the system (100) is configured to detect a compatibility of the medical device with a communication standard based on a device information, wherein the system (100) is configured to select a second message scheme from the plurality of message schemes which is compatible with the communication standard.

9. The system of one of the previous claims, wherein the system (100) is configured to modify a message scheme of the plurality of message schemes based on information on a mapping of a device parameter to a system parameter.

10. The system of one of the previous claims, wherein the second device driver comprises a conversation flow loader configured to load the second message scheme from the plurality of message schemes.

11. The system of one of the previous claims, wherein the medical device is a POCT device.

12. The system of one of the previous claims, wherein the system (100) is configured to generate individual device drivers for a plurality of different medical devices based on the first device driver.

13. The system of one of the previous claims, wherein the system (100) is configured to transmit a message of the message type to the medical device according to the second message scheme for the message type using the second device driver.

14. A computer-implemented method (700) for generating a device driver for a medical device, wherein the method comprises:
receiving (710) a communication request or a response to a communication request from a medical device;
transmitting (720) a message of a message type to the medical device according to a first message scheme for the message type using a first device driver;
receiving (730) an error message from the medical device in response to the message;
selecting (740) a second message scheme for the message type from a plurality of message schemes; and
generating (750) a second device driver based on the first device driver and the second message scheme.

15. A computer program having a program code for performing a method according to claim 14 when the program is executed on a processor.
